# EUROPEAN PATENT APPLICATION

(11) **EP 4 663 575 A1**
(43) Date of publication of application: **17.12.2025**
(21) Application number: 24763777.0
(22) Date of filing: 22.02.2024
(51) Int. Cl.: B65D 81/20, A61J 1/14, A61J 1/18, B65B 55/02, B65B 55/08, B65D 81/26

(54) **PACKAGED BODY OF RUBBER PRODUCT FOR MEDICAL USE, AND METHOD FOR STERILIZING SAME**

(30) Priority: 28.02.2023 JP 2023029903
(60) Divisional application: 25201451.9
(71) Applicant: SUMITOMO RUBBER INDUSTRIES, LTD., Kobe-shi, Hyogo 651-0072 (JP)
(72) Inventor: YAMASE Keisuke, Kobe-shi, Hyogo 651-0072 (JP); KONDO Toshikazu, Kobe-shi, Hyogo 651-0072 (JP); KIDA Yogun, Kobe-shi, Hyogo 651-0072 (JP)
(74) Representative: Manitz Finsterwald Patent- und Rechtsanwaltspartnerschaft mbB
(86) International application number: PCT/JP2024/006508
(87) International publication number: WO 2024/181296

(57) **Abstract**

[Problem to be solved] An object of the present invention is to provide a novel package having a medical rubber product stored therein and capable of keeping the oxygen therein at a predetermined concentration or lower. Another object of the present invention is to make it possible to visually confirm the oxygen concentration in the package, thereby facilitating quality assurance by sterilization treatment.

[Solution to solve problem] The present invention is a package having a medical rubber product sealed therein, wherein the package is formed of an oxygen impermeable packaging material and has a first space section and a second space section, the first space section has the medical rubber product stored therein, the second space section has an oxygen detecting agent stored therein, the oxygen detecting agent changes a color tone depending upon an oxygen concentration, and the first space section and the second space section are divided by an oxygen permeable divider.

## Description

### TECHNICAL FIELD

The present invention relates to sterilization treatment of a medical rubber product, and more specifically relates to a package of a medical rubber product and a sterilization treatment method thereof.

### BACKGROUND ART

A medical rubber stopper for sealing an opening of a syringe, a vial or the like is required to have many properties such as non-elution, high cleanliness, chemical resistance, resistance to needle piercing, self-sealability and high slidability. The quality of the characteristics required for the medical rubber stopper should comply with the regulations stipulated in "Test for Rubber Closure for Aqueous Infusions" of the 17th edition of the Japanese Pharmacopoeia in terms of use of the medical rubber stopper.

There is an increasing demand for a medical product (such as a syringe gasket or a vial stopper) to be delivered in a sterilized state, i.e. to be ready to use (RTU). Sterilization assurance methods include a high-pressure steam sterilization, an ethylene oxide gas (EOG) sterilization, and a radiation sterilization.

When the medical rubber product is treated by the EOG sterilization, a process for removing EOG is required due to concerns about EOG residues. When the medical rubber product is treated by the high-pressure steam sterilization, time for drying steam is required. A gamma ray sterilization, which is a type of the radiation sterilization, can sterilize the medical rubber product in a state of being packaged and thus has an advantage that the medical rubber product can be delivered without opening the package. Therefore, there is a tendency of applying the gamma ray sterilization for the medical rubber product.

As the technologies relating to the radiation sterilization treatment, Patent literatures 1 and 2 can be exemplified.

Patent literature 1 discloses a method for sterilizing a plastic-containing article by radioactive ray or electron beam irradiation, comprising sealing the plastic-containing article together with a deoxidizing agent in a substantially gas impermeable bag or container and deoxidizing the gas impermeable bag or container, followed by irradiating the gas impermeable bag or container with a radioactive ray or electron beam.

Patent literature 2 discloses a packaging material for radiation sterilization treatment formed from a laminate having an odor adsorption layer, wherein the odor adsorption layer is formed from a resin composition containing an odor adsorbent formed by carrying a chemical adsorbent on an inorganic porous material.

### Citation List

### Patent Literature

Patent literature 1: JP H08-89561 A
Patent literature 2: JP 2014-233408 A

### SUMMARY OF THE INVENION

### Technical Problem

When a medical rubber product is sterilized by gamma ray irradiation, the cleavage and crosslinking of the polymer constituting the medical rubber product occur at the same time. Absorption of excessive gamma ray promotes the cleavage of the backbone of the polymer constituting the medical rubber product, resulting in the formation of low-molecular components. Therefore, the elution performance of the medical rubber product after the gamma ray sterilization deteriorates. In addition, the cleaved low-molecular components bleed out of the surface of the rubber product, and the medical rubber products adhere to each other, causing trouble of clogging the part feeder used in the manufacturing process of medical products.

One option is to add an antioxidant to the medical rubber product to absorb the excessive gamma ray. However, there are concerns that the addition of the antioxidant may impair the elution characteristics and adversely affect the pharmaceuticals.

In view of the above problems, the applicant has found that a medical rubber product maintaining non-elution performance can be obtained by reducing the oxygen concentration in the package having the medical rubber product stored therein and subjecting the package to the gamma ray sterilization treatment, and has filed patent applications (Japanese Patent Applications No. 2021-208609 and No. 2022-169472). However, it is difficult to confirm for each package whether the oxygen concentration in the package before sterilization treatment and the package before shipment is a predetermined value or lower, and there is a problem in quality assurance.

The present invention has been made in view of the above circumstances, and an object of the present invention is to make it possible to visually confirm the oxygen concentration in the package, thereby facilitating quality assurance by sterilization treatment.

### Solution to Problem

The package according to the present invention is a package having a medical rubber product sealed therein, wherein the package is formed of an oxygen impermeable packaging material and has a first space section and a second space section, the first space section has the medical rubber product stored therein, the second space section has an oxygen detecting agent stored therein, the oxygen detecting agent changes a color tone depending upon an oxygen concentration, and the first space section and the second space section are divided by an oxygen permeable divider.

The package according to the present invention is a secondary package having a primary package stored therein, wherein the primary package has a medical rubber product sealed therein, the primary package is formed of an oxygen impermeable packaging material and has a first space section, a second space section and a third space section, the first space section has the medical rubber product stored therein, the second space section has an oxygen detecting agent stored therein, the third space section has a deoxidizing agent and/or an oxygen detecting agent stored therein, the oxygen detecting agent changes a color tone depending upon an oxygen concentration, the first space section and the second space section are divided by an oxygen permeable divider, the first space section and the third space section are divided by an oxygen impermeable divider, and an air vent communicating the third space section with an internal space of the secondary package is formed through the primary package.

The present invention includes a sterilization method of the package according to the present invention.

### Effect of the invention

According to the present invention, the oxygen concentration in the package can be visually confirmed, thereby facilitating quality assurance by sterilization treatment.

### BRIEF DESCRIPTION OF THE DRAWINGS

Fig. 1 is an explanatory drawing schematically showing one embodiment of the package of the medical rubber product according to the present invention,
Fig. 2 is an explanatory drawing schematically showing another embodiment of the package of the medical rubber product according to the present invention, and
Fig. 3 is an explanatory drawing schematically showing another embodiment of the package of the medical rubber product according to the present invention.

### DESCRIPTION OF EMBODIMENTS

### <Package>

The package according to the present invention is a package having a medical rubber product sealed therein, wherein the package is formed of an oxygen impermeable packaging material and has a first space section and a second space section, the first space section has the medical rubber product stored therein, the second space section has an oxygen detecting agent stored therein, the oxygen detecting agent changes a color tone depending upon an oxygen concentration, and the first space section and the second space section are divided by an oxygen permeable divider.

The package according to the present invention is a package having the packaging materials in the form of a film stacked on each other, the peripheral edges thereof being sealed to form a storage section inside thereof. Examples of the form of the package include a four-side sealed type and a three-side sealed type.

With respect to the four-side sealed package, two packaging materials in the form of the film with the same shape and size are stacked on each other and peripheral edge seal parts are formed on three surrounding sides and an opening is formed on the remaining side. After the contents are filled and stored through the opening, the opening is sealed to seal the package.

With respect to the three-side sealed package, one packaging material in the form of the film is folded in half along a folding line, and the resultant areas divided by the folding line is made the front and back films, and peripheral edge seal parts are formed on the two sides. The remaining side forms an opening, and after the contents are filled and stored through the opening, the opening is sealed to seal the package.

The shape of the package according to the present invention in a planar view is not particularly limited, and is preferably approximately rectangular, more preferably rectangular. The length of one side of the approximate rectangle is preferably 17 cm or more, more preferably 20 cm or more, and is preferably 75 cm or less, more preferably 52 cm or less.

The package according to the present invention has a first space section having the medical rubber product stored therein, and a second space section having an oxygen detecting agent stored therein, wherein the oxygen detecting agent changes a color tone depending upon an oxygen concentration. The first space section and the second space section are divided by an oxygen permeable divider.

The package according to the present invention has an up-and-down direction and a width direction perpendicular to the up-and-down direction. The upper side of the package is a side where the opening is formed for inserting the medical rubber product, the deoxidizing agent and/or the oxygen detecting agent, and the like, and is a side that is sealed after these contents are stored in the package.

The package according to the present invention preferably has at least an upper edge seal part and a side edge seal part, and may have an upper edge seal part, a side edge seal part, and a lower edge seal part. The peripheral edge seal parts of the package are preferably formed by thermal welding (heat sealing).

The peripheral edge seal parts of the package are preferably provided linearly along the peripheral edge of the package, more preferably provided in a straight line. The sealing width of the peripheral edge seal part of the package is preferably 0.05 mm or more, more preferably 0.1 mm or more, and even more preferably 0.5 mm or more, and is preferably 5.0 mm or less, more preferably 3.0 mm or less, and even more preferably 1.0 mm or less. If the width of the peripheral edge seal part of the package is 0.05 mm or more, the package has enhanced airtightness. If the width of the peripheral edge seal part of the package is 5.0 mm or less, the storage space inside the package can be increased.

The divider between the first space section and the second space section is preferably provided in a manner of bridging the upper edge seal part and one side edge seal part of the package. The shape of the divider between the first space section and the second space section is not particularly limited, and is preferably linear, more preferably curved or bent. The upper edge seal part of the first space section and the upper edge seal part of the second space section are preferably adjacent in the width direction to form the upper edge seal part of the package. The side edge seal part of the first space section and the side edge seal part of the second space section are preferably adjacent in the up-and-down direction to form one side edge seal part.

The second space section is divided by the upper edge seal part, the side edge seal part and the divider to form a space for storing the oxygen detecting agent that changes the color tone depending upon the oxygen concentration.

The first space section is divided by the upper edge seal part, the side edge seal parts, the lower edge of the package and the divider to form a space for storing the medical rubber product.

The divider between the first space section and the second space section preferably has an air vent through which the medical rubber product and the oxygen detecting agent are not traversable and by which the first space section and the second space section are oxygen permeable.

The divider between the first space section and the second space section is preferably provided with the air vent by discontinuously welding (heat sealing) the oxygen impermeable packaging material by heat. The first space section and the second space section communicate with each other through the air vent. The width of the seal part forming the divider between the first space section and the second space section is preferably 0.3 mm or more, more preferably 0.5 mm or more, and even more preferably 1.0 mm or more, and is preferably 3.0 mm or less, more preferably 2.5 mm or less, and even more preferably 2.0 mm or less.

The number of the air vent provided in the divider between the first space section and the second space section is not particularly limited, and is preferably 1 or more, more preferably 3 or more, and is preferably 10 or less, more preferably 5 or less. The width of one air vent is preferably 1 mm or more, more preferably 3.0 mm or more, and is preferably 15 mm or less, more preferably 10 mm or less. If the number and width of the air vent hole are set as described above, the gas permeability between the first space section and the second space section is good. In addition, if the width of the air vent is set within the above range, it is possible to prevent the oxygen detecting agent changing the color tone depending upon the oxygen concentration stored in the second space section from moving to the first space section.

In the embodiment that the oxygen detecting agent is stored in the second space section, the oxygen detecting agent detects the oxygen concentration in the first space section, since the first space section and the second space section communicate with each other. The color tone of the oxygen detecting agent changes in accordance with the oxygen concentration, and thus the oxygen concentration in the first space section of the package can be visually recognized.

A deoxidizing agent may be stored together with the oxygen detecting agent in the second space section, and in this embodiment, the oxygen concentration in the first space section and the second space section can be reduced by the deoxidizing agent. For example, it is possible to remove oxygen remaining in an internal space in a package having the internal space filled with an inert gas and sealed, or oxygen permeating from the outside of the package into the first space section.

The package according to the present invention preferably further has a third space section for storing a deoxidizing agent and/or an oxygen detecting agent.

Unlike the divider between the first space section and the second space section, the divider between the first space section and the third space section is preferably provided without the air vent so as to prevent the permeation of oxygen. The divider between the first space section and the third space section is preferably a seal part formed by continuously welding (heat sealing) the oxygen impermeable packaging material by heat.

The divider between the first space section and the third space section is provided in a manner of bridging the upper edge seal part and one side edge seal part. The shape of the divider between the first space section and the third space section is not particularly limited, and is preferably linear, more preferably curved or bent.

The third space section is divided by the upper edge seal part, the side edge seal part and the divider to form a space for storing the deoxidizing agent and/or the oxygen detecting agent changing the color tone depending upon the oxygen concentration.

The width of the seal part forming the divider between the first space section and the third space section is preferably 0.3 mm or more, more preferably 0.5 mm or more, and even more preferably 1.0 mm or more, and is preferably 3.0 mm or less, more preferably 2.5 mm or less, and even more preferably 2.0 mm or less. If the width of the seal part of the divider is 0.3 mm or more, the third space section has enhanced airtightness. If the width of the seal part of the divider is 3.0 mm or less, the storage space of the third space section can be increased.

The upper edge seal part of the first space section is located between the upper edge seal part of the second space section and the upper edge seal part of the third space section. The upper edge seal part of the first space section, the upper edge seal part of the second space section and the upper edge seal part of the third space section are adjacent in the width direction to form the upper edge seal part of the package. The side edge seal part of the third space section and the side edge seal part of the first space section are adjacent in the up-and-down direction to form one side edge seal part.

The third space section may be provided with an air vent communicating with the outside of the package. The air vent is preferably provided in such a size that the deoxidizing agent and/or the oxygen detecting agent changing the color tone depending upon the oxygen concentration cannot protrude. The air vent is preferably provided in the side edge seal part of the third space section.

Examples of the embodiment in which the package according to the present invention has the second space section and the third space section include a first embodiment in which the oxygen detecting agent is stored in the second space section and the deoxidizing agent is stored in the third space section; a second embodiment in which the oxygen detecting agent is stored in the second space section and the deoxidizing agent and the oxygen detecting agent are stored in the third space section; a third embodiment in which the oxygen detecting agent and the deoxidizing agent are stored in the second space section and the deoxidizing agent is stored in the third space section; and a fourth embodiment in which the oxygen detecting agent and the deoxidizing agent are stored in the second space section and the deoxidizing agent and the oxygen detecting agent are stored in the third space section.

The oxygen concentration in the internal space of the package according to the present invention is preferably 1.0 vol % or less, more preferably 0.5 vol % or less, and even more preferably 0.1 vol % or less. This is because if the oxygen concentration in the package is 1.0 vol % or less, deterioration of the rubber caused by gamma ray irradiation is suppressed, and the non-elution characteristic of the medical rubber product after gamma ray sterilization treatment becomes better.

The internal space of the package according to the present invention is preferably filled with an inert gas. If the internal space is filled with the inert gas, the oxygen concentration in the package decreases. Thus, the amount of the deoxidizing agent used can be reduced, or the desired oxygen concentration can be achieved without using the deoxidizing agent. Further, even when the deoxidizing agent is used, the amount of decrease in the internal pressure of the package caused by the oxygen absorption of the deoxidizing agent can be reduced, and thus adhesion between products caused by the decrease in the internal pressure can be prevented. Examples of the inert gas include a rare gas such as helium, neon and argon, and nitrogen gas. The internal space of the package according to the present invention is preferably filled with nitrogen.

### <Secondary package>

The present invention includes a package that is a secondary package having a primary package stored therein, wherein the primary package has a medical rubber product sealed therein, the primary package is formed of an oxygen impermeable packaging material and has a first space section, a second space section and a third space section, the first space section has the medical rubber product stored therein, the second space section has an oxygen detecting agent stored therein, the third space section has a deoxidizing agent and/or an oxygen detecting agent stored therein, the oxygen detecting agent changes a color tone depending upon an oxygen concentration, the first space section and the second space section are divided by an oxygen permeable divider, the first space section and the third space section are divided by an oxygen impermeable divider, and an air vent communicating the third space section with an inner space of the secondary package is formed through the primary package.

Details of the primary package stored in the secondary package according to the present invention are as described above and explanation about them is omitted.

The secondary package is not particularly limited, as long as it is formed of an oxygen impermeable packaging material.

The secondary package may be formed of a packaging material that is same as or different from the packaging material constituting the primary package.

The secondary package is preferably obtained by making a bag from the packaging material in the form of a film. With respect to the secondary package, packaging materials in the form of the film are stacked on each other, and the peripheral edge thereof is sealed to form a storage section inside. Examples of the form of the package include a four-side sealed type and a three-side sealed type.

The shape of the secondary package in a planar view is not particularly limited, and is preferably approximately rectangular, more preferably rectangular. Further, from the viewpoint of storing a plurality of primary packages, the length of one side of the approximate rectangle of the secondary package is preferably 30 cm or more, more preferably 50 cm or more, and is preferably 100 cm or less, more preferably 85 cm or less.

Examples of the embodiment in which the primary package stored in the secondary package according to the present invention has the second space section and the third space section include a first embodiment in which the oxygen detecting agent is stored in the second space section and the deoxidizing agent is stored in the third space section; a second embodiment in which the oxygen detecting agent is stored in the second space section and the deoxidizing agent and the oxygen detecting agent are stored in the third space section; a third embodiment in which the oxygen detecting agent and the deoxidizing agent are stored in the second space section and the deoxidizing agent is stored in the third space section; and a fourth embodiment in which the oxygen detecting agent and the deoxidizing agent are stored in the second space section and the deoxidizing agent and the oxygen detecting agent are stored in the third space section.

The secondary package according to the present invention may store the primary package of only one embodiment of the above-mentioned embodiments, or may store the primary package of any combination of the above-mentioned embodiments. In addition, the number of the primary package stored in the secondary package is not particularly limited, as long as it is 1 or more. The number of the primary package stored in the secondary package is more preferably 2 or more, and is preferably 10 or less, more preferably 4 or less.

The internal space of the secondary package according to the present invention is preferably filled with an inert gas. If the internal space is filled with the inert gas, the oxygen concentration in the secondary package decreases. Thus, the amount of the deoxidizing agent used can be reduced. Examples of the inert gas include a rare gas such as helium, neon and argon, and nitrogen gas. The internal space of the package according to the present invention is preferably filled with nitrogen.

The internal space of the secondary package according to the present invention may be filled with air, as long as the oxygen concentration in the internal space of the primary package is controlled to a predetermined value or lower (preferably 1.0 vol % or less). This is because if the oxygen concentration in the internal space of the primary package is controlled to the predetermined value or lower (preferably 1.0 vol % or less), lowering in the non-elution characteristic of the medical rubber product caused by gamma ray sterilization treatment can be suppressed. Further, this is because there is no need to fill the secondary package with the inert gas, which improves the production efficiency.

In the embodiment that the oxygen detecting agent is stored in the third space section of the primary package, the oxygen detecting agent detects the oxygen concentration in the internal space of the secondary package since the third space section and the internal space of the secondary package communicate with each other. The color tone of the oxygen detecting agent changes in accordance with the oxygen concentration, and thus the oxygen concentration in the internal space of the secondary package can be visually recognized.

In the embodiment that the deoxidizing agent is stored in the third space section of the primary package, the deoxidizing agent absorbs the oxygen in the internal space of the secondary package since the third space section and the internal space of the secondary package communicate with each other. In this embodiment, the oxygen concentration in the internal space of the secondary package can be reduced by the deoxidizing agent. For example, when the internal space of the secondary package is filled with the air and sealed, if the oxygen concentration in the internal space of the secondary package is reduced by the deoxidizing agent, the permeation of the oxygen from the internal space of the secondary package to the first space section of the primary package can be prevented. Further, when the internal space of the secondary package is filled with the inert gas and sealed, if the oxygen remaining in the internal space of the secondary package is removed, the permeation of the oxygen from the internal space of the secondary package to the first space section of the primary package can be reliably prevented.

It is also preferable to further store the secondary package according to the present invention in a package (e.g. a tertiary package to a quinary package). The tertiary package to the quinary package is not particularly limited, as long as it can be irradiated with gamma ray. Examples of the tertiary package to the quinary package include a bag and a box. Examples of the packaging bag include a packaging bag formed of a thermoplastic resin film made of polyethylene, polyamide, polyester or the like, or a packaging bag formed of aluminum. The packaging bag is preferably sealable. The packaging box is not particularly limited, and examples thereof include a paper box and a cardboard box.

### <Material constituting package>

The package according to the present invention is formed of an oxygen impermeable packaging material. Regarding the oxygen permeability (ml/(m²·24h·atm)) of the oxygen impermeable packaging material used in the present invention at 20 °C and 90% RH, the primary package is preferably 10000 ml/(m²·24h·atm) or less, more preferably 1000 ml/(m²·24h·atm) or less, and even more preferably 20 ml/(m²·24h·atm) or less, and the secondary package is preferably 1000 ml/(m²·24h·atm) or less, more preferably 200 ml/(m²·24h·atm) or less, and even more preferably 20 ml/(m²·24h·atm) or less. This is because if the packaging material having the oxygen permeability in the above range is used, the oxygen concentration in the package can be controlled.

The oxygen impermeable packaging material used in the present invention is preferably capable of being thermal welded (heat sealed). This is because the packaging material can be easily made into a bag by thermal welding (heat sealing). In addition, the packaging material is preferably a transparent or translucent material such that a change in the color tone of the oxygen detecting agent can be confirmed.

The oxygen impermeable packaging material used in the present invention is preferably a laminated film. The laminated film preferably has, for example, a base layer, a barrier layer, a sealant layer, or the like.

Examples of the base layer include a resin film. Examples of the resin component of the resin film include polyethylene, polypropylene, cyclic polyolefin, fluorine-based resin, polystyrene, acrylonitrile-styrene copolymer (AS resin), acrylonitrile-butadiene-styrene copolymer (ABS resin), polyvinyl chloride, fluorine-based resin, poly(meth)acrylic resin, polycarbonate, polyester such as polyethylene terephthalate (PET) and polyethylene naphthalate, polyamide such as various nylon, polyimide, polyamideimide, polyaryl phthalate, silicone resin, polysulfone, polyphenylene sulfide, polyether sulfone, polyurethane, acetal resin, and cellulose-based resin.

As the base layer, for example, an unstretched nylon film, a stretched nylon film, a PET film, a linear low-density polyethylene film, or a low-density polyethylene film is preferable.

The base layer preferably includes a film using one or at least two resins selected from the above-mentioned resins, and may be composed of one layer or at least two layers having the same or different composition.

As the base layer, a single layered or multilayered film formed by a film forming method such as an extrusion method, a cast molding method, a T-die method, a cutting method and an inflation method can be used. In addition, the thickness of the base layer can be appropriately determined by those skilled in the art, and is preferably 6 µm or more, more preferably 9 µm or more, and is preferably 150 µm or less, more preferably 130 µm or less.

The barrier layer is a layer to prevent the invasion of water vapor or oxygen from the outside into the inside of the package.

The barrier layer may be, for example, a metal foil or a transparent gas barrier film. Examples of the metal foil include a foil made of pure aluminum or aluminum alloy. Examples of the transparent gas barrier film include a resin film made of polyolefin, a vinyl-based polymer, polyester, polyamide or the like provided with a deposited inorganic film or an organic coating film.

Examples of the deposited inorganic film include an aluminum vapor deposition film, a silica vapor deposition film, an alumina vapor deposition film, and a silica/alumina binary vapor deposition film. Examples of the organic coating film include a polyvinylidene chloride coating film and a polyvinylidene fluoride coating film.

For example, the thickness of the barrier layer is preferably 5 µm or more, more preferably 7 µm or more, and is preferably 30 µm or less, more preferably 10 µm or less in order to provide appropriate strength to the package and ensure processability such as heat sealing.

The sealant layer is a layer to impart the thermal welding (heat sealing) property to the packaging material. As the material of the sealant layer, a polyolefin-based resin is generally used of thermoplastic resins. Specifically, an ethylenebased resin such as low-density polyethylene resin (LDPE), medium-density polyethylene resin (MDPE), linear low-density polyethylene resin (LLDPE), ethylenevinyl acetate copolymer (EVA), ethylene-α olefin copolymer, and ethylenemethacrylic acid resin copolymer; a blend resin of polyethylene and polybutene; and a polypropylene-based resin such as homopolypropylene resin (PP), propylene-ethylene random copolymer, propylene-ethylene block copolymer, propylene-α olefin copolymer can be used. In addition, a cycloolefin polymer (COP) or a cycloolefin copolymer (COC) can also be used.

As the packaging material used in the present invention, the base layer, the barrier layer and the sealant layer may be laminated by forming an adhesive layer between respective layers. Examples of the adhesive layer include an EC (extrusion coat) layer, and a layer made of an adhesive for dry lamination, an adhesive for non-solvent lamination, or the like.

When the adhesive layer is laminated using the extrusion coat, it can be formed by extrusion coating an adhesive or adhesive composition on a layer film to be adhered, although there is no particular limitation. During the extrusion coating, the adhesive or adhesive composition is firstly heated and melted, and expanded and stretched in the required width direction with a T-die to be extruded into a curtain shape, and the melt is allowed to flow down onto the layer film to be adhered, and is sandwiched between a rubber roll and a cooled metal roll, thereby simultaneously forming the adhesive layer and adhering and laminating it onto the layer film to be adhered.

When the adhesive for dry lamination is used for the adhesive layer, the adhesive dispersed or dissolved in a solvent is applied to one film and dried, the other film is overlapped and laminated thereon, and then the adhesive is aged at a temperature of 30 °C to 120 °C for several hours to several days, thereby curing and laminating the adhesive.

When the adhesive for non-solvent lamination is used, the adhesive itself which is not dispersed or dissolved in a solvent is applied to a film and dried, the other film is overlapped and laminated thereon, and then the adhesive is aged at a temperature of 30 °C to 120 °C for several hours to several days, thereby curing and laminating the adhesive.

These adhesives may be heat-curable adhesives, ultraviolet-curable adhesives, electron beam-curable adhesives, or the like. Examples of such an adhesive include a polyvinyl acetate-based adhesive such as polyvinyl acetate and vinyl acetate-ethylene copolymer, a polyacrylic acid-based adhesive made of a copolymer of polyacrylic acid and polystyrene, polyester, polyvinyl acetate, etc., a cyanoacrylate-based adhesive, an ethylene copolymer-based adhesive made of a copolymer of ethylene and a monomer such as vinyl acetate, ethyl acrylate, acrylic acid and methacrylic acid, a cellulose-based adhesive, a polyurethane-based adhesive, a polyester-based adhesive, a polyamide-based adhesive, a polyimidebased adhesive, a polyolefin-based adhesive, an amino resin-based adhesive made of a urea resin, a melamine resin or the like, a phenol resin-based adhesive, an epoxy-based adhesive, a reactive (meth)acrylic adhesive, an elastomer adhesive made of chloroprene rubber, nitrile rubber, styrene-butadiene rubber or the like, a silicone-based adhesive, and an inorganic adhesive made of an alkali metal silicate, a low-melting point glass or the like.

In addition, the adhesive may be any type such as an aqueous type, a solution type, an emulsion type and a dispersion type. Further, the adhesive may be in any state such as a film/sheet state, a powder state and a solid state. Furthermore, the adhesive mechanism of the adhesive may be any one such as a chemical reaction type, a solvent evaporation type, a thermal melting type and a thermal pressing type.

The adhesive layer is formed by applying the adhesive by, for example, roll coating, gravure roll coating or kiss coating, and the coating amount is preferably about 0.1 g/m² to 10 g/m² (dry state). If the coating amount of the adhesive falls within the above range, good adhesion can be obtained.

The thickness of the oxygen impermeable packaging material used in the present invention is not particularly limited, and is preferably 10 µm or more, more preferably 20 µm or more, and even more preferably 40 µm or more, and is preferably 300 µm or less, more preferably 200 µm or less, and even more preferably 150 µm or less. This is because if the thickness of the packaging material falls within the above range, the strength of the packaging material and the workability of the packaging operation are not impaired.

### <Oxygen detecting agent>

The oxygen detecting agent used in the present invention changes the color tone depending upon the oxygen concentration. This is because if the color tone of the oxygen detecting agent changes in accordance with the oxygen concentration, the oxygen concentration in the package can be visually confirmed.

The oxygen detecting agent used in the present invention is preferably capable of detecting an oxygen concentration in the package of 1.0 vol % or less, more preferably capable of detecting an oxygen concentration in the package of 0.5 vol % or less, and even more preferably capable of detecting an oxygen concentration in the package of 0.1 vol % or less. The lower the detectable concentration is, the easier the quality of the medical rubber product after the sterilization treatment is guaranteed.

Examples of the oxygen detecting agent include an oxidation-reduction pigment, a reducing agent, a binder made of a resin having both a hydrophilic group and a hydrophobic group, and one formed by applying an ink composition containing a solvent for an oxygen indicator to a support.

As the oxidation-reduction pigment, new methylene blue, neutral red, indigo carmine, safranine **T,** phenosafranine, Capri blue, Nile blue, diphenylamine, xylene cyanol, nitrodiphenylamine, ferroin, and N- phenylanthranilic acid can be used, in addition to methylene blue.

As the reducing agent, ascorbic acid, erythorbic acid and its salt, a salt of ascorbic acid, a reducing sugar such as D-arabinose, D-erythros, D-galactose, D-xylose, D-glucose, D-mannose, D-fructose and D-lactose, and a metal salt such as stannous salt and ferrous salt, etc. can be used.

The concentration of oxygen in the surrounding atmosphere can be detected not only by the change in the hue of the oxidation-reduction pigment, but also by the change in the hue of the mixed color with a pigment having another color. For this purpose, a colorant can be added to the ink composition for the oxygen indicator.

Examples of the colorant to be added include a red colorant such as Food Red No. 2 (Amaranth), Food Red No. 3 (Erythrosine), Food Red No. 40 (Allura Red AC), Food Red No. 102 (New Coccine), Food Red No. 104 (Phloxine), Food Red No. 106 (Acid Red), and natural cochineal pigment, a yellow colorant such as Food Yellow No. 4 (Tartrazine), Food Yellow No. 5 ((Sunset Yellow FCF), and natural safflower yellow pigment, and a blue colorant such as Food Blue No. 1 (Brilliant Blue FCF), and Food Blue No. 2 (Indigo Carmine).

The binder is used to fix the oxidation-reduction pigment, reducing agent or colorant on the support, and is preferably a resin having both a hydrophilic group and a hydrophobic group. Specific examples include a polyvinyl acetal resin, methylcellulose, ethylcellulose, and a polyester resin with an introduced hydrophilic group, and the polyvinyl acetal resin is particularly preferable.

The solvent is preferably one that can uniformly and stably dissolve or disperse the oxidation-reduction pigment, reducing agent, colorant and especially one that can dissolve or disperse the binder. Specifically, aromatic hydrocarbons, aliphatic hydrocarbons, esters, alcohols, water or the like can be used.

The support is preferably one that does not react with the ink composition for the oxygen indicator and does not inhibit the color development of the oxidation-reduction pigment. Specifically, paper, synthetic paper, nonwoven fabric, a synthetic resin film or the like can be used.

Specific examples of the oxygen detecting agent include AGELESS EYE (registered trademark) (available from Mitsubishi Gas Chemical Co., Ltd.). For example, the AGELESS EYE is blue when the oxygen concentration is 0.5 vol % or more, and is pink when the oxygen concentration is 0.10 vol % or less.

### <Deoxidizing agent>

The deoxidizing agent used in the present invention is not particularly limited, as long as it has ability of absorbing oxygen. As the deoxidizing agent, a known deoxidizing agent can be used, examples thereof include a deoxidizing agent using sulfite, bisulfite, dithionite, hydroquinone, catechol, resorcin, pyrogallol, gallic acid, rongalite, ascorbic acid and/or a salt thereof, isoascorbic acid and/or a salt thereof, sorbose, glucose, lignin, dibutylhydroxytoluene, butylhydroxyanisole, an oxygen absorbing base agent of such metal powder as ferrous salt, and iron powder, or the like, and is appropriately selected according to the purpose.

In addition, if necessary, one or at least two metal halides such as sodium chloride, potassium chloride, magnesium chloride, calcium chloride, aluminum chloride, ferrous chloride, ferric chloride, sodium bromide, potassium bromide, magnesium bromide, calcium bromide, iron bromide, nickel bromide, sodium iodide, potassium iodide, magnesium iodide, calcium iodide and iron iodide may be added as an oxidation catalyst into the metal powder-based deoxidizing agent.

The deoxidizing agent may contain moisture or a moisture donor where necessary, and may also contain a deodorizer, deodorant, or another filler. In addition, the shape of the deoxidizing agent is not particularly limited, and the deoxidizing agent may be, for example, a powder, granule, block, sheet, or the like, and may be a sheet or film-like deoxidizing agent having various oxygen absorbing compositions dispersed in a thermoplastic resin.

Specific examples of the deoxidizing agent used in the present invention include AGELESS (registered trademark) (available from Mitsubishi Gas Chemical Co., Ltd.).

### <Production method of package>

The package according to the present invention can be obtained by making a bag from the packaging material. Specifically, it is produced as follows.

One packaging material is folded or two packaging materials are stacked on each other such that the sealant layers (heat sealing surfaces) of the packaging material face to each other, and the peripheral edge parts thereof are thermally welded (heat sealed). At this time, the upper edge part among the peripheral edge parts is not thermally welded (heat sealed) to produce a bag having an opening formed for storing the medical rubber product and the like.

The dividing line part forming the divider between the first space section and the second space section is thermally welded (heat sealed). The divider between the first space section and the second space section is discontinuously thermally welded (heat sealed) to form an air vent according to the desired shape of the divider. The area that is not thermally welded (heat sealed) forms the air vent. It is noted that the thermal welding (heat sealing) for forming the divider between the first space section and the second space section may be carried out simultaneously with the step of thermally welding (heat sealing) the peripheral edge parts of the package.

When the third space section is formed in the package, the divider between the first space section and the third space section is continuously thermally welded (heat sealed) such that the first space section and the third space section do not communicate with each other according to the desired shape of the divider. The thermal welding (heat sealing) for forming the divider between the first space section and the third space section may be carried out simultaneously with the step of thermally welding (heat sealing) the peripheral edge parts of the package.

The conditions for the thermal welding (heat sealing) can be appropriately changed depending on the packaging material used, and for example, the following conditions are preferably adopted.

The temperature is preferably from 130 °C to 160 °C, more preferably from 140 °C to 150 °C.

The time is preferably from 1 second to 10 seconds, more preferably from 2 seconds to 5 seconds.

Next, the medical rubber product is stored in the first space section of the bag, and the oxygen detecting agent changing the color tone depending upon the oxygen concentration is stored in the second space section of the bag.

When the third space section is formed in the package, the deoxidizing agent and/or the oxygen detecting agent having the color tone that changes in accordance with the oxygen concentration is store in the third space section of the bag.

The bag having the medical rubber product stored therein is set in a heat sealer, and the inside of the bag is replaced with an inert atmosphere. Specifically, the oxygen in the bag is sucked out and the bag is filled with an inert gas. Examples of the inert gas include a rare gas such as helium, neon and argon, and nitrogen gas.

Next, the upper edge of the bag is heat sealed and the opening of the bag is sealed to obtain the package according to the present invention having the medical product sealed therein.

In the present invention, as the heat sealing method, for example, a known method such as a bar sealing, a rotating roll sealing, a belt sealing, an impulse sealing, a high-frequency sealing, and an ultrasonic sealing can be applied.

### [Medical rubber product]

Examples of the medical rubber product to be packaged in the present invention include a sliding or sealing part, such as a rubber stopper or sealing member of a container (e.g. a vial) for various pharmaceuticals such as a liquid preparation, a powder preparation and a lyophilized preparation, a rubber stopper for a vacuum blood collection tube, a plunger stopper for a prefilled syringe, and a nozzle cap.

The medical rubber product is preferably formed from a medical rubber composition containing (a) a rubber component. As (a) the rubber component, a butyl rubber is preferable, and a halogenated butyl rubber is more preferable, from the viewpoint of chemical resistance and resistance to gas permeation. Examples of the halogenated butyl rubber include a chlorinated butyl rubber, a brominated butyl rubber, and a bromide of a copolymer of isobutylene and p-methylstyrene. As the halogenated butyl rubber, the chlorinated butyl rubber or brominated butyl rubber is preferable. The chlorinated butyl rubber or brominated butyl rubber is, for example, obtained by an addition reaction or substitution reaction between chlorine or bromine and an isoprene moiety in a butyl rubber, specifically a double bond and/or a carbon atom adjacent to a double bond of the isoprene moiety. It is noted that the butyl rubber is a copolymer obtained by polymerizing isobutylene and a small amount of isoprene.
(a) The rubber component may contain a rubber component other than the butyl rubber. Examples of the other rubber components include an isoprene rubber, a butadiene rubber, a styrene-butadiene rubber, a natural rubber, a chloroprene rubber, a nitrile-based rubber such as an acrylonitrile-butadiene rubber, a hydrogenated nitrile-based rubber, a norbornene rubber, an ethylene-propylene rubber, an ethylene-propylene-diene rubber, an acrylic rubber, an ethylene-acrylate rubber, a fluorine rubber, a chlorosulfonated polyethylene rubber, an epichlorohydrin rubber, a silicone rubber, a urethane rubber, a polysulfide rubber, a phosphanzene rubber, and a 1,2-polybutadiene rubber. These rubbers may be used solely, or two or more of these rubbers may be used in combination.

In the case that the other rubber component is used, the amount of the butyl rubber in (a) the rubber component is preferably 90 mass % or more, more preferably 95 mass % or more, and even more preferably 98 mass % or more. In addition, it is also preferable that (a) the rubber component consists of the halogenated butyl rubber.

The medical rubber composition preferably contains (b) a crosslinking agent. (b) The crosslinking agent is added for crosslinking (a) the rubber component. (c) The crosslinking agent is not particularly limited, as long as it is a crosslinking agent capable of crosslinking the rubber. Examples of (c) the crosslinking agent include sulfur, a metal oxide, a resin crosslinking agent, an organic peroxide, and a triazine derivative. These crosslinking agents may be used solely, or two or more of these crosslinking agents may be used in combination.

The amount of (b) the crosslinking agent in the medical rubber composition is preferably 0.2 part by mass or more, more preferably 0.4 part by mass or more, and even more preferably 0.6 part by mass or more, and is preferably 20 parts by mass or less, more preferably 15 parts by mass or less, and even more preferably 10 parts by mass or less, with respect to 100 parts by mass of (a) the rubber component. This is because if the amount of (b) the crosslinking agent falls within the above range, a rubber having better rubber physical properties (hardness, tensile properties, Cset) and processability (little burn) can be obtained.

The medical rubber composition preferably does not contain a vulcanization accelerator. This is because the vulcanization accelerator may remain in the final rubber product, and dissolve into the pharmaceutical liquid in a vial. Examples of the vulcanization accelerator include a guanidine accelerator (e.g. diphenylguanidine), a thiuram accelerator (e.g. tetramethylthiuram disulfide, tetramethylthiuram monosulfide), a dithiocarbamate accelerator (e.g. zinc dimethyldithiocarbamate), a thiazole accelerator (e.g. 2-mercaptobenzothiazole, dibenzothiazyl disulfide), and a sulfenamide accelerator (e.g. N-cyclohexyl-2-benzothiazole sulfenamide, N-t-butyl-2-benzothiazole sulfenamide).

The medical rubber composition may contain a (c) a hydrotalcite. (c) The hydrotalcite functions as an anti-scorching agent when crosslinking the halogenated butyl rubber, and also functions as an agent to prevent increase in compression permanent strain of the medical rubber member. Further, the hydrotalcite also functions as an acid acceptor to absorb chlorine-based gas or bromine-based gas generating when crosslinking the halogenated butyl rubber and prevent occurrence of crosslinking inhibition caused by these gases. It is noted that the above-mentioned magnesium oxide is also capable of functioning as an acid acceptor.

Examples of the hydrotalcite include one or at least two members selected from a Mg-Al hydrotalcite such as Mg_{4.5}Al₂(OH)₁₃CO₃·3.5H₂O, Mg_{4.5}Al₂(OH)₁₃CO₃, Mg₄Al₂(OH)₁₂CO₃·3.5H₂O, MgsAl₂(OH)₁₆CO₃·4H₂O, Mg₅Al₂(OH)₁₄CO₃·4H₂O, and Mg₃Al₂(OH)₁₀CO₃·1.7H₂O.

The medical rubber composition may further contain (d) a filler. Examples of (d) the filler include an inorganic filler such as clay and talc, and a resin powder of an olefin resin, a styrene elastomer and an ultra-high molecular weight polyethylene (UHMWPE). Among them, as the filler, the inorganic filler is preferable, and the clay or talc is more preferable. The filler functions as adjusting the rubber hardness of the medical rubber member, and also functions as a weight increasing material to reduce the production cost of the medical rubber component.

The amount of (d) the filler in the medical rubber composition is preferably appropriately set according to the desired rubber hardness or the like of the medical rubber component. For example, the amount of (d) the filler in the medical rubber composition is preferably 5 parts by mass or more, more preferably 10 parts by mass or more, and even more preferably 20 parts by mass or more, and is preferably 200 parts by mass or less, more preferably 150 parts by mass or less, and even more preferably 100 parts by mass or less, with respect to 100 parts by mass of (a) the rubber component.

The medical rubber composition may further contain a coloring agent such as titanium oxide and carbon black, a lubricant such as stearic acid and low-density polyethylene (LDPE), polyethylene glycol acting as a crosslinking activator, a plasticizer (e.g. paraffin oil), or the like, in an appropriate proportion.

### [Sterilization method]

The package according to the present invention having the medical rubber product sealed therein is preferably sterilized by radioactive ray radiation, more preferably sterilized by gamma ray or electron beam irradiation. In the sterilization method, the package having a plurality of medical rubber products stored therein is preferably irradiated with radioactive ray.

Examples of the radioactive ray used in the sterilization treatment include α ray (helium nuclei), β ray (electron beam) and γ ray (gamma ray). The β ray (electron beam) has an extremely high dose rate (tens of thousands of times that of γ ray), so the sterilization treatment time is short, but its penetrating power is small because it is a particle beam. On the other hand, the gamma ray has a large penetrating power, but its dose rate is smaller than that of the electron beam, so the treatment time is long. In the present invention, it is preferable to use the gamma ray for the sterilization treatment, from the viewpoint of sterilizing the package having a plurality of medical rubber products stored therein.

Examples of the gamma ray include gamma ray radiated from cobalt 60, cesium 137 or the like, and the gamma ray radiated from cobalt 60 is preferable.

In the gamma ray irradiation, the gamma ray absorption dose of the medical rubber product is established in the actual sterilization validation procedure. In many cases, 15 kGy is adopted as the minimum absorption dose for ordinary medical devices. The irradiation dose of gamma ray required to ensure that the gamma ray absorption dose of all the medical rubber products in the package is 15 kGy or more varies depending on the number, placement way or the like of the medical rubber products in the package, and generally the irradiation dose is in a range from 1.4 times or more of 15 kGy and 2.0 times or less of 15 kGy. Similarly, when 20 kGy is adopted as the minimum absorption dose, the irradiation dose is in a range from 1.4 times or more of 20 kGy and 2.0 times or less of 20 kGy, and when 25 kGy is adopted as the minimum absorption dose, the irradiation dose is in a range from 1.4 times or more of 25 kGy and 2.0 times or less of 25 kGy. It is noted that the gamma ray absorption dose can be confirmed by attaching a dosimeter to the object to be irradiated.

In the sterilization method of the medical rubber product according to the present invention, the package having the oxygen detecting agent stored in the second space section thereof and being in a state that the oxygen concentration detected by the oxygen detecting agent is lower than a predetermined value is preferably irradiated with the gamma ray. The oxygen in the package storing the medical rubber product before the gamma ray irradiation is preferably reduced to a predetermined concentration or less. The oxygen concentration in the package is preferably 5 vol % or less, more preferably 3 vol % or less, and even more preferably 1 vol % or less. This is because if the oxygen concentration in the package is 5 vol % or less, deterioration of the medical rubber product caused by the gamma ray irradiation can be suppressed.

Examples of the method for reducing the oxygen concentration in the package to 5 vol % or less include a method of replacing the air in the package with an inert gas, and a method of storing a deoxidizing agent in the package.

Examples of the inert gas include a rare gas such as helium, neon and argon, and nitrogen gas.

The package (primary package, secondary package) storing the medical rubber product according to the present invention may be further stored in a package and irradiated with gamma ray. Examples of the package include a bag and a box. Examples of the packaging bag include a packaging bag formed from a thermoplastic resin film made of polyethylene, polyamide, polyester or the like, or a packaging bag formed from aluminum. The packaging bag is preferably sealable. The packaging box is not particularly limited, and examples thereof include a paper box and a cardboard box.

Examples of the package include a gas permeable package and a gas impermeable (gas sealable) package, and it is also preferable to use them in combination.

It is noted that when irradiating the package with gamma ray, the package having a plurality of medical rubber products stored therein is preferably irradiated with the gamma ray, for example, while the package is stored in a storage container made of aluminum alloy.

The sterilized medical rubber product according to the present invention preferably has a viable bacteria count (cfu: colony forming unit: number of colonies that appear when cultured) of 0 in a bioburden measurement test.

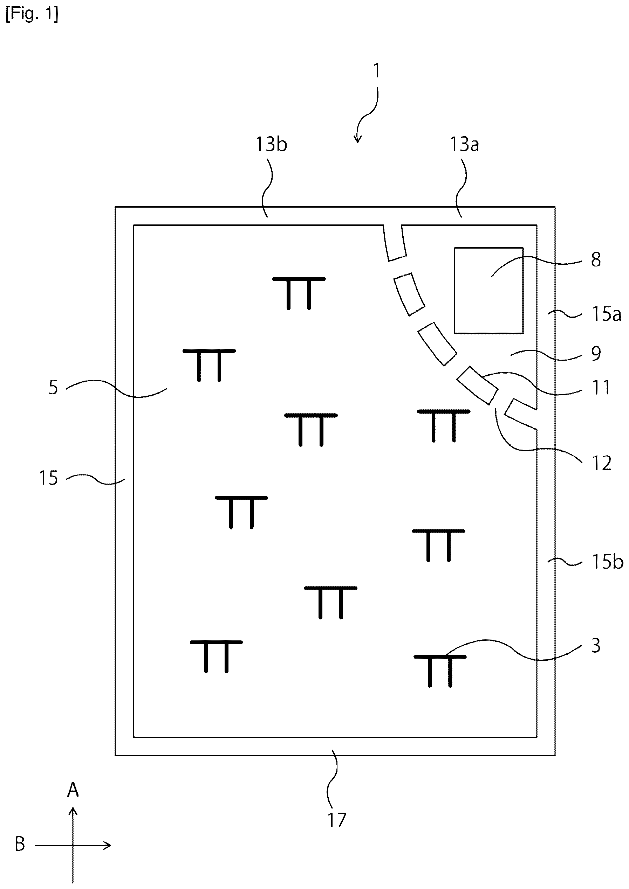

Next, the present invention will be further explained with reference to drawings, but the present invention is not limited to the embodiments shown in the drawings. Fig. 1 is a planar view explaining one embodiment of the package according to the present invention. The package 1 has an up-and-down direction A and a width direction B perpendicular to the up-and-down direction.

The package 1 is approximately rectangular in a planar view. The package 1 is a four-side sealed type. Two oxygen impermeable packaging materials are laminated, and the peripheral edges thereof are sealed by thermal welding (heat sealing). The package 1 has an upper edge seal part 13, side edge seal parts 15, and a lower edge seal part 17.

The package 1 has a first space section 5 having a medical rubber product 3 stored therein, and a second space section 9 having an oxygen detecting agent 8 stored therein, wherein the oxygen detecting agent 8 changes a color tone depending upon an oxygen concentration. The first space section 5 and the second space section 9 are divided by an oxygen permeable divider 11.

The second space section 9 is divided by an upper edge seal part 13a, a side edge seal part 15a and the divider 11 to form a space for storing the oxygen detecting agent 8 changing the color tone depending upon the oxygen concentration.

The first space section 5 is divided by an upper edge seal part 13b, side edge seal parts 15,15b, the lower edge seal part 17 and the divider 11 to form a space for storing the medical rubber product.

The divider 11 between the first space section 5 and the second space section 9 is provided in a curved shape in a manner of bridging the upper edge seal part 13 and one side edge seal part 15.

The upper edge seal part 13b of the first space section and the upper edge seal part 13a of the second space section 9 are adjacent in the width direction to form the upper edge seal part 13 of the package 1. The side edge seal part 15a of the second space section and the side edge seal part 15b of the first space section are adjacent in the up-and-down direction to form an edge seal part 15 on one side of the package.

The divider 11 between the first space section 5 and the second space section 9 is provided with a plurality of air vents 12 having a width of 10.0 mm by discontinuously thermally welding (heat sealing) the oxygen impermeable packaging material. The first space section 5 and the second space section 9 communicate with each other by the air vents 12.

In an embodiment that the oxygen detecting agent 8 is stored in the second space section 9, the oxygen detecting agent 8 detects the oxygen concentration in the first space section 5, since the first space section 5 and the second space section 9 communicate with each other. The oxygen detecting agent changes the color tone in accordance with the oxygen concentration, and thus the oxygen concentration in the first space section 5 of the package 1 can be visually recognized.

In an embodiment that a deoxidizing agent 7 together with the oxygen detecting agent 8 is stored in the second space section 9, the oxygen concentration in the first space section 5 and the second space section 9 can be reduced by the deoxidizing agent 7, since the first space section 5 and the second space section 9 communicate with each other.

The internal space of the package 1 is filled with nitrogen.

The three-side sealed package has the same configuration as the package in Fig. 1 except that it has no lower edge seal part 17, and thus explanation about it is omitted.

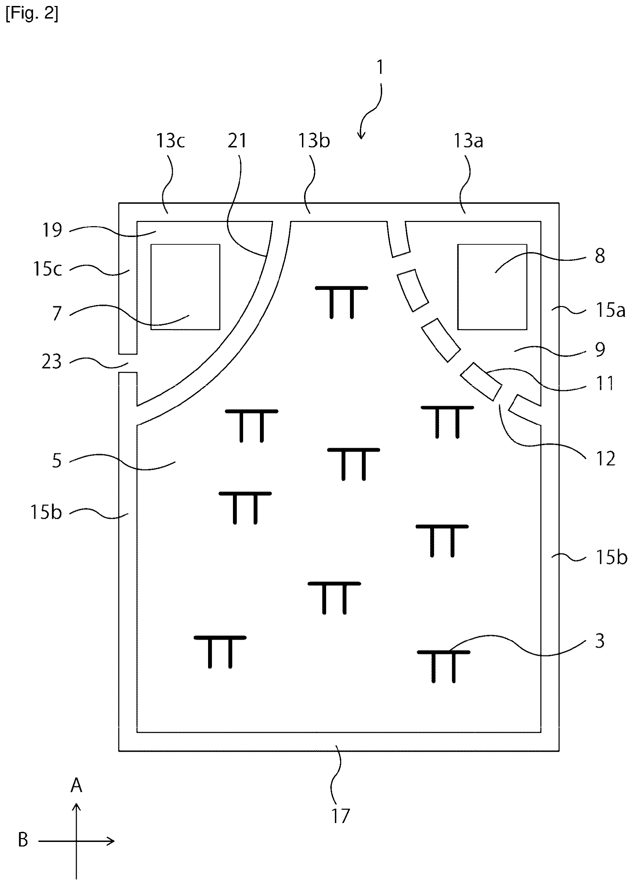

Fig. 2 is a planar view explaining another embodiment of the package according to the present invention. The package 1 has a first space section 5 having a medical rubber product 3 stored therein, a second space section 9 having an oxygen detecting agent 8 stored therein, and a third space section 19 having a deoxidizing agent 7 stored therein, wherein the oxygen detecting agent 8 changes a color tone depending upon an oxygen concentration.

The first space section 5 and the second space section 9 are divided by an oxygen permeable divider 11.

The second space section 9 is divided by an upper edge seal part 13a, a side edge seal part 15a and the divider 11 to form a space for storing the oxygen detecting agent 8 changing the color tone in accordance with the oxygen concentration.

The divider 11 between the first space section 5 and the second space section 9 is provided in a curved shape in a manner of bridging an upper edge seal part 13 and one side edge seal part 15.

The divider 11 between the first space section 5 and the second space section 9 is provided with a plurality of air vents 12 having a width of 10 mm by discontinuously thermally welding (heat sealing) the oxygen impermeable packaging material. The first space section 5 and the second space section 9 communicate with each other by the air vents 12.

The oxygen detecting agent 8 is stored in the second space section 9. The oxygen detecting agent 8 detects the oxygen concentration in the first space section 5, since the first space section 5 and the second space section 9 communicate with each other. The oxygen detecting agent 8 changes the color tone depending upon the oxygen concentration, and thus the oxygen concentration in the first space section 5 of the package 1 can be visually recognized.

The first space section 5 and the third space section 19 are divided by a divider 21. Unlike the divider 11 between the first space section 5 and the second space section 9, the divider 21 between the first space section 5 and the third space section 19 is not provided with the air vent so as to avoid permeation of oxygen. The divider 21 is preferably formed by continuously thermally welding (heat sealing) the oxygen impermeable packaging material.

The third space section 19 is divided by an upper edge seal part 13c, a side edge seal part 15c and the divider 21 to form a space for storing a deoxidizing agent and/or an oxygen detecting agent changing a color tone depending upon the oxygen concentration.

The first space section 5 is defined by an upper edge seal portion 13b, side edge seal portions 15b,15b, a lower edge seal portion 17 and the partitions 11, 21 to form a space for storing the medical rubber product.

The partition 21 between the first space section 5 and the third space section 19 is provided in a curved shape in a manner of spanning from the upper edge seal portion 13 to one side edge seal portion 15.

The upper edge seal part 13b of the first space section 5 is located between the upper edge seal part 13a of the second space section 9 and the upper edge seal part 13c of the third space section 19. The upper edge seal part 13b of the first space section 5, the upper edge seal part 13a of the second space section 9 and the upper edge seal part 13c of the third space section 19 are adjacent in the width direction to form the upper edge seal part 13 of the package 1. The side edge seal part 15a of the second space section 9 and the side edge seal part 15b of the first space section 5 are adjacent in the up-and-down direction to form an edge seal part 15 on one side. The side edge seal part 15c of the third space section 19 and the side edge seal part 15b of the first space section 5 are adjacent in the up-and-down direction to form an edge seal part 15 on another side.

The side edge seal part 15c of the third space section 19 is provided with an air vent 23. The air vent 23 is preferably provided in such a size that the deoxidizing agent and/or the oxygen detecting agent changing the color tone depending upon the oxygen concentration cannot protrude.

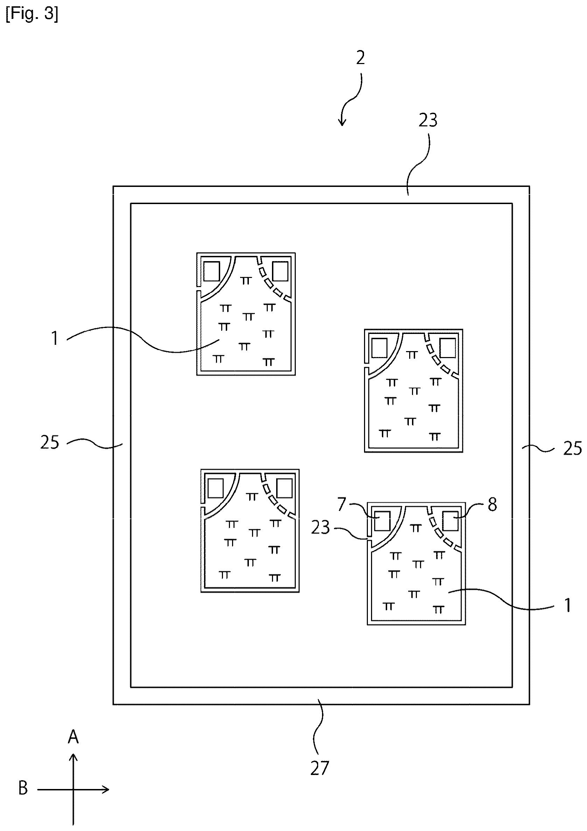

Fig. 3 is an explanatory drawing explaining an embodiment of a secondary package 2 storing a plurality of packages 1 of the embodiment in Fig. 2 as the primary package.

The secondary package 2 according to the present invention is approximately rectangular in a planar view. The secondary package 2 is a four-side sealed type. Two oxygen impermeable packaging materials are laminated, and the peripheral edges thereof are sealed by thermal welding (heat sealing). The secondary package 2 has an upper edge seal part 23, a side edge seal part 25, and a lower edge seal part 27. The peripheral edge seal parts of the secondary package 2 are provided in a straight line along the peripheral edges of the secondary package.

The deoxidizing agent 7 is stored in the third space section 19 of the primary package 1. If the side edge seal part 15c of the third space section 19 of the primary package 1 is provided with the air vent 23, when the primary packages 1 are stored in the secondary package 2, the oxygen concentration in the internal space of the secondary package 2 that is the outside of the primary package 1 can be reduced.

Fig. 3 shows an embodiment in which the oxygen detecting agent 8 is stored in the second space section 9 and the deoxidizing agent 7 is stored in the third space section 19 of the primary package 1, but the oxygen detecting agent 8 may be stored in the third space section 19. In this embodiment, the oxygen detecting agent 8 stored in the third space section 19 of the primary package 1 can detect the oxygen concentration in the internal space of the secondary package 2.

### EXAMPLE

Next, the present invention will be described in detail by way of examples. However, the present invention is not limited to the examples described below. Various changes and modifications without departing from the spirit of the present invention are included in the scope of the present invention.

### [Production of package of medical rubber product]

### (Example 1)

As a packaging material, a film having a length of 20 cm and a width of 17 cm (trade name: Suntech LD, oxygen permeability: 7900 ml/(m²·24h·atm)) available from Asahi Kasei Corporation was folded, and the side edge parts thereof were thermally welded (heat sealed) to produce a bag having an open upper edge. At the same time as the side edge parts were heat sealed, the divider between the first space section and the second space section, the divider between the first space section and the third space section were formed by thermal welding (heat sealing). The divider between the first space section and the second space section is discontinuously thermally welded (heat sealed) to form one air vent having a width of 10 mm. The divider between the first space section and the third space section is continuously thermally welded (heat sealed) to produce an oxygen impermeable divider without an air vent.

Rubber stoppers for a vial were stored as a medical rubber product in the first space section, AGELESS EYE available from Mitsubishi Gas Chemical Co., Ltd. was stored as an oxygen detecting agent in the second space section, and AGELESS available from Mitsubishi Gas Chemical Co., Ltd. was stored as a deoxidizing agent in the third space section. As the rubber stopper for the vial, a rubber stopper for a lyophilized injection vial having a flange diameter of 19.0 mm, a total height of 13.0 mm, a leg part diameter of 7.20 mm and a flange puncture part thickness of 2.5 mm was used.

The bag having the medical rubber product, oxygen detecting agent and deoxidizing agent stored therein was set in a heat sealer. The oxygen in the bag was sucked out and nitrogen was filled in, the upper edge of the bag was thermally welded (heat sealed), and the bag was sealed to produce a primary package. It is noted that the filling of nitrogen into the bag was conducted by inserting a degassing pipe and a nitrogen filling pipe into the bag and repeating a cycle of degassing and then filling with nitrogen three times.

The side edge seal part of the third space section of the primary package was cut to produce an air vent communicating the third space section with the outside.

As a packaging material of the secondary package, a laminated film having a length of 50 cm and a width of 40 cm (trade name: NJK, oxygen permeability: 49 ml/(m²·24h·atm)) available from Kurilon Kasei Co., Ltd. was used. The packaging material was folded and stacked on each other such that the sealant layers faced to each other, and the side edge portions thereof were heat sealed to produce a bag having an open upper edge. One primary package was stored in the bag having the open upper edge, and set in a heat sealer. The upper edge of the bag was thermally welded (heat sealed) to seal the bag. It is noted that the primary package was stored in the bag without replacing inside thereof with nitrogen, so air existed inside the secondary package and outside the primary package immediately after the storage.

The obtained secondary package was irradiated with gamma ray such that the absorbed gamma ray dose of the medical rubber product was 25 kGy, to carry out the sterilization treatment.

### (Evaluation result of Example 1)

The primary package of Example 1 has the second space section and the third space section. The oxygen detecting agent was stored in the second space section and the deoxidizing agent was stored in the third space section. The oxygen in the air inside the secondary package was absorbed by the deoxidizing agent in the third space section. The color tone of the oxygen detecting agent in the second space section of the primary package before the gamma ray sterilization treatment had a pink color, and thus it was confirmed that the oxygen concentration in the primary package was 0.1 vol % or less.

After the gamma ray sterilization treatment, the medical rubber products were removed from the primary package, and subjected to an elution test. The result was good.

### (Example 2)

Instead of filling the secondary package with air in the above Example 1, a degassing pipe and a nitrogen filling pipe were inserted into the bag for the secondary package, and a cycle of degassing and then filling with nitrogen was repeated three times. Then, the obtained secondary package was irradiated with gamma ray such that the absorbed gamma ray dose of the medical rubber product was 25 kGy, to carry out the sterilization treatment. In Example 2, the color tone of the oxygen detecting agent in the second space section of the primary package before the gamma ray sterilization treatment also had a pink color, and thus it was confirmed that the oxygen concentration in the primary package was 0.1 vol % or less. Further, after the gamma ray sterilization treatment, the medical rubber products were removed from the primary package, and subjected to an elution test. The result was good.

### (Example 3)

As a packaging material, a film having a length of 20 cm and a width of 17 cm (trade name: Suntech LD, oxygen permeability: 7900 ml/(m²·24h·atm)) available from Asahi Kasei Corporation was folded, and the side edge parts thereof were thermally welded (heat sealed) to produce a bag having an open upper edge. As the same time as the side edge parts were heat sealed, the divider between the first space section and the second space section was formed by thermal welding (heat sealing). The divider between the first space section and the second space section is discontinuously thermally welded (heat sealed) to form one air vent having a width of 10 mm.

Rubber stoppers for a vial were stored as a medical rubber product in the first space section, and AGELESS EYE available from Mitsubishi Gas Chemical Co., Ltd. was stored as an oxygen detecting agent in the second space section. As the rubber stopper for the vial, a rubber stopper for a lyophilized injection vial having a flange diameter of 19.0 mm, a total height of 13.0 mm, a leg part diameter of 7.20 mm and a flange puncture part thickness of 2.5 mm was used.

The bag having the medical rubber product and oxygen detecting agent stored therein was set in a heat sealer. The oxygen in the bag was sucked out and nitrogen was filled in, the upper edge of the bag was thermally welded (heat sealed), and the bag was sealed to produce a primary package. It is noted that the filling of nitrogen into the bag was conducted by inserting a degassing pipe and a nitrogen filling pipe into the bag and repeating a cycle of degassing and then filling with nitrogen three times.

As a packaging material of the secondary package, a laminated film having a length of 50 cm and a width of 40 cm (trade name: NJK, oxygen permeability: 49 ml/(m²·24h·atm)) available from Kurilon Kasei Co., Ltd. was used. The packaging material was folded and stacked on each other such that the sealant layers faced to each other, and the side edge parts thereof were heat sealed to produce a bag having an open upper edge. One primary package was stored in the bag having the open upper edge, and set in a heat sealer. The oxygen in the bag was sucked out and nitrogen was filled in, and the upper edge of the bag was thermally welded (heat sealed) to seal the bag. It is noted that the filling of nitrogen into the bag was conducted by inserting a degassing pipe and a nitrogen filling pipe into the bag and repeating a cycle of degassing and then filling with nitrogen three times.

The obtained secondary package was irradiated with gamma ray such that the absorbed gamma ray dose of the medical rubber product was 25 kGy, to carry out the sterilization treatment.

### (Evaluation result of Example 3)

The primary package of Example 3 has the second space section having the oxygen detecting agent stored therein. The color tone of the oxygen detecting agent in the second space section before the gamma ray sterilization treatment had a pink color, and thus it was confirmed that the oxygen concentration in the primary package was 0.1 vol % or less.

After the gamma ray treatment, the medical rubber products were removed from the primary package, and subjected to an elution test. The result was good.

### Industrial Applicability

According to the present invention, the oxygen concentration in the package can be visually confirmed, thereby facilitating quality assurance by sterilization treatment.

The preferable embodiment (1) according to the present invention is a package having a medical rubber product sealed therein, wherein
the package is formed of an oxygen impermeable packaging material and has a first space section and a second space section,
the first space section has the medical rubber product stored therein,
the second space section has an oxygen detecting agent stored therein,
the oxygen detecting agent changes a color tone depending upon an oxygen concentration, and
the first space section and the second space section are divided by an oxygen permeable divider.

The preferable embodiment (2) according to the present invention is the package according to the embodiment (1), wherein the divider has an air vent through which the medical rubber product and the oxygen detecting agent are not traversable and by which the first space section and the second space section are oxygen permeable.

The preferable embodiment (3) according to the present invention is the package according to the embodiment (1) or (2), wherein the divider between the first space section and the second space section is formed by discontinuously welding the packaging material by heat so as to provide the air vent.

The preferable embodiment (4) according to the present invention is the package according to any one of the embodiments (1) to (3), wherein an upper edge seal part of the first space section and an upper edge seal part of the second space section are adjacent.

The preferable embodiment (5) according to the present invention is the package according to the embodiment (4), wherein the upper edge seal part of the first space section and the upper edge seal part of the second space section are respectively sealed by thermal welding.

The preferable embodiment (6) according to the present invention is the package according to any one of the embodiments (1) to (5), wherein an internal space of the package is filled with nitrogen.

The preferable embodiment (7) according to the present invention is the package according to any one of the embodiments (1) to (6), wherein the second space section has a deoxidizing agent together with the oxygen detecting agent stored therein.

The preferable embodiment (8) according to the present invention is a secondary package having a primary package stored therein, wherein
the primary package has a medical rubber product sealed therein,
the primary package is formed of an oxygen impermeable packaging material and has a first space section, a second space section and a third space section,
the first space section has the medical rubber product stored therein,
the second space section has an oxygen detecting agent stored therein,
the third space section has a deoxidizing agent and/or an oxygen detecting agent stored therein,
the oxygen detecting agent changes a color tone depending upon an oxygen concentration,
the first space section and the second space section are divided by an oxygen permeable divider,
the first space section and the third space section are divided by an oxygen impermeable divider, and
an air vent communicating the third space section with an internal space of the secondary package is formed thorough the primary package.

The preferable embodiment (9) according to the present invention is the package according to the embodiment (8), wherein the divider between the first space section and the second space section has an air vent through which the medical rubber product and the oxygen detecting agent are not traversable and by which the first space section and the second space section are oxygen permeable.

The preferable embodiment (10) according to the present invention is the package according to the embodiment (8) or (9), wherein the divider between the first space section and the second space section is formed by discontinuously welding the packaging material by heat so as to provide the air vent.

The preferable embodiment (11) according to the present invention is the package according to the embodiment (8) or (9), wherein an upper edge seal part of the first space section is adjacent to an upper edge seal part of the second space section and an upper edge seal part of the third space section.

The preferable embodiment (12) according to the present invention is the package according to any one of the embodiments (8) to (11), wherein the upper edge seal parts of the first space section, the second space section and the third space section are sealed by thermal welding.

The preferable embodiment (13) according to the present invention is the package according to any one of the embodiments (8) to (12), wherein an internal space of the primary package and an internal space of the secondary package are filled with nitrogen.

The preferable embodiment (14) according to the present invention is a sterilization method of a medical rubber product, comprising a step of irradiating the package according to any one of the embodiments (1) to (7) with gamma ray.

The preferable embodiment (15) according to the present invention is the sterilization method of the medical rubber product according to the embodiment (14), wherein the package having the oxygen detecting agent stored in the second space section thereof and being in a state that the oxygen concentration detected by the oxygen detecting agent is lower than a predetermined value is irradiated with the gamma ray.

The preferable embodiment (16) according to the present invention is a sterilization method of a medical rubber product, comprising a step of irradiating the package according to any one of the embodiments (8) to (13) with gamma ray.

The preferable embodiment (17) according to the present invention is the sterilization method of the medical rubber product according to the embodiment (16), wherein the package having the oxygen detecting agent stored in the second space section thereof and being in a state that the oxygen concentration detected by the oxygen detecting agent is lower than a predetermined value is irradiated with the gamma ray.

### Reference Signs List

1: package (primary package), 2: secondary package, 3: medical rubber product, 5: first space section, 7: deoxidizing agent, 8: oxygen detecting agent, 9: second space section, 11: partition, 13: upper edge seal portion, 15: side edge seal portion, 17: lower edge seal portion, 19: third space section, 21: partition

## Claims

1. A package having a medical rubber product sealed therein, wherein
the package is formed of an oxygen impermeable packaging material and has a first space section and a second space section,
the first space section has the medical rubber product stored therein,
the second space section has an oxygen detecting agent stored therein,
the oxygen detecting agent changes a color tone depending upon an oxygen concentration, and
the first space section and the second space section are divided by an oxygen permeable divider.

2. The package according to claim 1, wherein the divider has an air vent through which the medical rubber product and the oxygen detecting agent are not traversable and by which the first space section and the second space section are oxygen permeable.

3. The package according to claim 2, wherein the divider between the first space section and the second space section is formed by discontinuously welding the packaging material by heat so as to provide the air vent.

4. The package according to claim 1, wherein an upper edge seal part of the first space section and an upper edge seal part of the second space section are adjacent.

5. The package according to claim 4, wherein the upper edge seal part of the first space section and the upper edge seal part of the second space section are respectively sealed by thermal welding.

6. The package according to claim 1, wherein an internal space of the package is filled with nitrogen.

7. The package according to claim 1, wherein the second space section has a deoxidizing agent together with the oxygen detecting agent stored therein.

8. A secondary package having a primary package stored therein,
wherein
the primary package has a medical rubber product sealed therein,
the primary package is formed of an oxygen impermeable packaging material and has a first space section, a second space section and a third space section,
the first space section has the medical rubber product stored therein,
the second space section has an oxygen detecting agent stored therein,
the third space section has a deoxidizing agent and/or an oxygen detecting agent stored therein,
the oxygen detecting agent changes a color tone depending upon an oxygen concentration,
the first space section and the second space section are divided by an oxygen permeable divider,
the first space section and the third space section are divided by an oxygen impermeable divider, and
an air vent communicating the third space section with an internal space of the secondary package is formed through the primary package.

9. The package according to claim 8, wherein the divider between the first space section and the second space section has an air vent through which the medical rubber product and the oxygen detecting agent are not traversable and by which the first space section and the second space section are oxygen permeable.

10. The package according to claim 9, wherein the divider between the first space section and the second space section is formed by discontinuously welding the packaging material by heat so as to provide the air vent.

11. The package according to claim 8, wherein an upper edge seal part of the first space section is adjacent to an upper edge seal part of the second space section and an upper edge seal part of the third space section.

12. The package according to claim 11, wherein the upper edge seal parts of the first space section, the second space section and the third space section are sealed by thermal welding.

13. The package according to claim 8, wherein an internal space of the primary package and an internal space of the secondary package are filled with nitrogen.

14. A sterilization method of a medical rubber product, comprising a step of irradiating the package according to claim 1 with gamma ray.

15. The sterilization method of the medical rubber product according to claim 14, wherein the package having the oxygen detecting agent stored in the second space section thereof and being in a state that the oxygen concentration detected by the oxygen detecting agent is lower than a predetermined value is irradiated with the gamma ray.

16. A sterilization method of a medical rubber product, comprising a step of irradiating the package according to claim 8 with gamma ray.

17. The sterilization method of the medical rubber product according to claim 16, wherein the package having the oxygen detecting agent stored in the second space section thereof and being in a state that the oxygen concentration detected by the oxygen detecting agent is lower than a predetermined value is irradiated with the gamma ray.
